# EUROPEAN PATENT APPLICATION

(11) **EP 1 782 730 A1**
(43) Date of publication of application: **09.05.2007**
(21) Application number: 05425771.2
(22) Date of filing: 02.11.2005
(51) Int. Cl.: A61B 5/0404

(54) **Device for acquiring, recording and transmitting ECG signals for home care needs**

(71) Applicant: Hosand Technologies S.r.l., 28924 Verbania (IT)
(72) Inventor: Lopez Torres, Fernando, 08292 Esparraguera-Barcelona (ES)
(74) Representative: Petruzziello, Aldo

(57) **Abstract**

A device (100) for acquiring, recording and transmitting electrocardiographic (ECG) signals in relation to homecare needs is described. The device comprises a box (1, 2) containing a printed circuit card (4) whereon is mounted a processor (5), a memory (51) and a transmission device (6). Two recessed seats (21, 21') are formed in the box wherein are disposed two sensors (22, 22') designed to detect the electrical signal indicative of the ECG through contact of a finger of the right hand and a finger of the left hand of the patient. The sensors (22, 22') are connected electrically to the processor (5) to store in the memory (51) the ECG data detected and transmit them by means of the transmitter (6) to a doctor's remote acquisition device, for consultation thereof.

## Description

The present invention relates to a device for acquiring, recording and transmitting electrocardiographic (ECG) signals in relation to home care needs, integrated for preventive, diagnostic or rehabilitation purposes.

Acquisition of the electrocardiographic signal from a patient normally takes place through an electrocardiograph of considerable size, to which can be connected a number of electrodes (minimum two) which allow the electrocardiographic tracing from the leads to be acquired through the difference in potential that is generated by the electrical activity of the heart.

Normally, in a healthcare institution, the tracing is acquired through twelve curves (called leads) corresponding to the electrical signals generate by pairs of electrodes. These electrodes are positioned at different points of the patient's body and made more effective through the use of a conductive gel which is applied to the patient's skin. Some electrocardiographs can generate twenty-four leads.

A complete diagnosis of the general state of health of the patient's heart can take place only through the complete analysis of all twelve leads. However, each lead provides different information. In particular the one known as lead 1, which is normally taken between the two electrodes positioned on the patient's wrists, provides indications relating to disturbances of heart rhythm, atrioventricular blocks, superventricular tachycardia, P-QRS ratio, arrhythmias and ventricular tachycardia.

The electrocardiogram can be read only by a specialist physician.

There exist on the market portable devices which allow only some leads to be detected and transmitted by fixed or mobile telephone to a cardiology centre qualified to read it.

However, these devices present the problem of positioning of the electrodes, which must be done precisely in order to pick up the correct signal.

Patients who present specific identified risks, or pathological phenomena that occur randomly, or patients who have been discharged but require continuous monitoring, are obliged either to prolong their hospital stay or else to report with a certain frequency to an outpatient's department to have an electrocardiogram. However, this method often does not produce the desired results in that some disorders of heart rhythm, which could be important signals for evaluation of the risk the patient runs, occur in an absolutely random manner.

Support apparatus (e.g. Holter) able to record the electrocardiographic tracing for 24 consecutive hours for subsequent analysis by a specialist physician are routinely used. These devices, though considerably increasing the probability of picking up pathological phenomena, do not, however, represent an exhaustive response to the problem of the random nature of the occurrence of some alterations.

The object of the present invention is to provide a device for detecting, recording and transmitting electrocardiographic signals which partially solves the problems set forth above.

Another object of the present invention is to provide such a device for detecting, recording and transmitting electrocardiographic signals that is simple for the user to use.

Another object of the present invention is to provide such a detecting, recording and transmitting device for electrocardiographic signals that is versatile and able to transmit the stored information to a physician or hospital centre in a rapid and simple manner.

These objects are achieved in accordance with the invention with the characteristics listed in appended independent claim 1.

Advantageous embodiments of the invention are apparent from the dependent claims.

The device for acquiring, recording and transmitting electrocardiographic signals (ECG) in relation to the homecare needs, according to the invention, comprises a box containing a printed circuit card on which are mounted a processor, a memory and a transmission device. Two recessed seats are formed inside the box in which are disposed two sensors able to detect the electrical signal indicative of the ECG through the contact of a finger of the right hand and a finger of the left hand of the patient. The sensors are connected electrically to the processor to store in the memory the ECG data recorded and transmit them, by means of the transmitter, to a physician's remote acquisition device for consultation thereof.

In particular, the device according to the invention does not use moveable electrodes, such as those of a common electrocardiograph, which require the presence of specialized personnel for their application. In fact the device according to the invention simply requires the use of a finger of the right hand and a finger of the left hand of the patient and it records, through the contact of the fingers with the sensors, the tracing from lead 1 of the ECG.

This means that the device can be used easily, even at home and even by elderly people or those with little aptitude for the use of technology, still providing, within the limits for which it has been invented, useful results for monitoring of patients at risk or patients in rehabilitation.

Once the ECG signal has been detected, since it is a device intended for home use, this ECG signal is stored in the memory of the device for possible later transfer, by means of a transmitter, to a special device of the physician, such as a PC, for examination and reporting.

The device according to the invention can transmit the signal detected and stored with different modalities: a) The patient takes the device with him when he goes to the doctor and the doctor himself downloads the tracing into his PC through an infrared port.

b) The device can be connected to a mobile or fixed telephone and the tracing recorded inside the device is transmitted either to the telephone or the PC of the doctor.

The device can be connected to a gateway able to perform other homecare functions, including video monitoring or voice connection. The tracing recorded is transmitted to this gateway which, according to procedures defined by the doctor and suitably configured, transmits it, via Intranet or Internet, to a client on which the doctor can observe it an possibly intervene.

Further characteristics of the invention will be made clearer by the detailed description that follows, referring to a purely exemplifiying and therefore non-limiting embodiment thereof, illustrated in the appended drawings, in which:
Figure 1 is a top plan view illustrating the device for acquiring, recording and transmitting ECG signals in relation to homecare needs, according to the invention;
Figure 2 is a side view of the device of Figure 1;
Figure 3 is cross sectional view, illustrating the device of Figure 1 partially exploded, and
Figure 4 is a block diagram illustrating diagrammatically operation of the device according to the invention.

The device for acquiring, recording and transmitting ECG signals in relation to homecare needs according to the invention, indicated as a whole with reference numeral 100, is described with the aid of the figures.

Hereinunder the term top indicates the surface of the device destined to face towards the user and the term bottom indicates that destined to face away from the user.

The device 100 is presented as a small-sized box comprising a bottom half-shell 1 and a top half-shell 2 which can be coupled together to enclose inside them a circuit card or board 4 on which is mounted a microprocessor or microcontroller 5 which controls operation of the device 100. The bottom half-shell 1 has a top peripheral edge 10 designed to engage in a snap coupling relationship with the bottom peripheral edge 20 of the top half-shell 2. The dimensions of the device 100 can vary according to the components used (as will be described later), but are in any case such as to make it pocket-sized.

Two round, concave, recessed seats 21 21' are formed on the top wall of the top half-shell 2, designed to receive a finger of the left hand and a finger of the right hand, respectively. Respective ECG signal detecting sensors 22, 22' which are connected by means of electrical wires 23, 23' to the circuit card 4 and to the microcontroller 5 are mounted in the recessed seats 21, 21'.

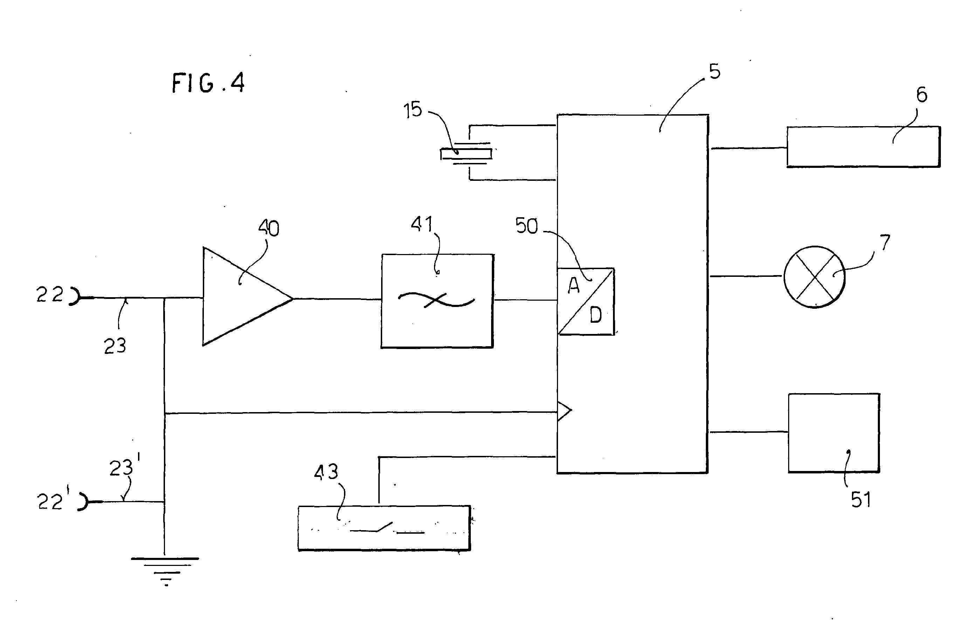

The sensors 22, 22' are electrodes able to detect the electrical signal generated by the heart's activity. As shown in Figure 4, the electrical signal detected by the sensors 22, 22' is amplified by an amplifier 40 and filtered by a filter 41 to obtain the ECG signal which is sent to the input of an analog-to-digital converter (ADC) 50 of the microprocessor 5. The amplifier 40 and the filter 41 are mounted on the circuit card 4; the ADC converter 50, on the other hand, is integrated into the microprocessor 5.

A memory 51, such as a flash memory, is connected to the microprocessor 5 to store the digital data indicative of the ECG signal coming from the ADC 50. The memory 51 can be integrated into the microprocessor 5 or external.

A crystal 15 which acts as a clock for the microcontroller timing functions is connected to the microcontroller 5.

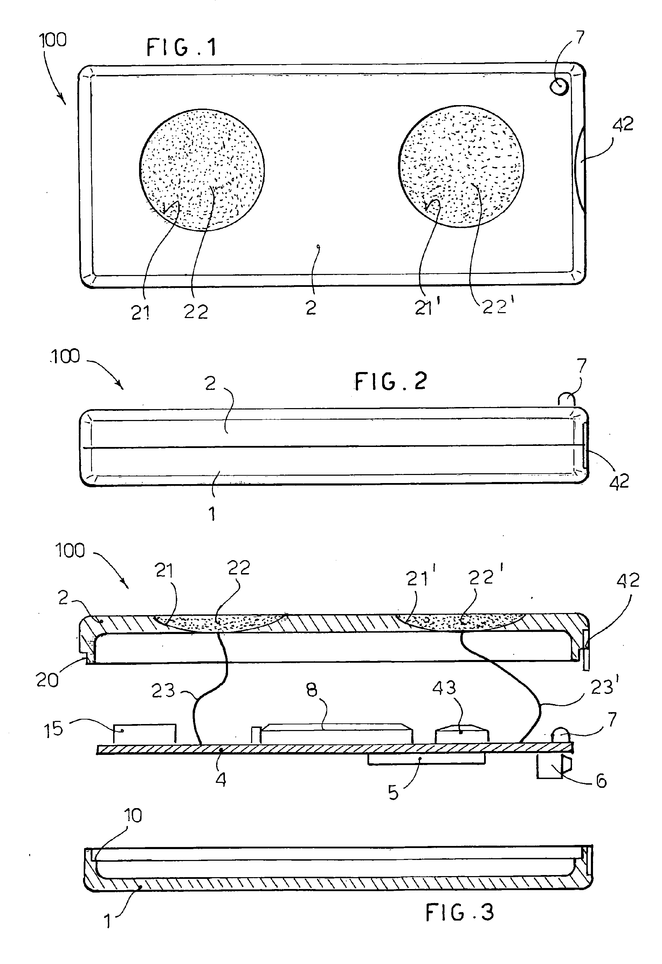

With reference also to Figure 3, a transmitter 6 is mounted on the circuit card 4 and is connected to the microprocessor 5 to transmit in wireless mode the digital data stored in the memory 51 to a complementary receiver of a remote acquisition device. The transmitter 6 can be any type of wireless transmitter.

The transmitter 6 is preferably an infrared transmitter which uses an IRDA protocol. In this manner the digital ECG data stored in the memory 51 can be transferred to a doctor's PC, provided with an IRDA receiver, so that the doctor can evaluate the ECG tracing inside his PC. The transmitter 6 can be disposed near one side of the top half-shell 2 and in register with a transparent window 42.

However, the transmitter 6 can be another type of transmitter, such as, for example, a wireless transmitter using WI-FI, Bluetooth, GSM or broad band telephony protocols. In this case the device 100 can be connected to a mobile or fixed telephone and the digital ECG data can be sent, over the telephone line, to the physician's telephone or computer.

Furthermore, by means of said wireless protocols, the device 100 can be connected to a gateway able to perform other homecare functions, including video monitoring or voice links with the patient. The ECG tracing recorded by the sensors 22, 22' and stored in the memory 51 is transmitted to this gateway which, according to the procedures defined by the physician and suitably configured, transmits it, by Intranet or Internet, to a client on which the physician can observe it and intervene if necessary.

A membrane switch 43 connected to the microprocessor 5 is mounted on the circuit card 4. The switch 43 is disposed beneath the sensor 22' of the right finger. Thus, the sensor 22' of the right finger, being mobile, by pressing on the membrane switch 43 also acts as a push button. The switch 43 has a dual function:
- a pushbutton for switching on the ECG signal detecting device, if a finger of the right hand and a finger of the left hand are in contact with the sensors 22, 22', and
- a pushbutton for enabling transmission of the stored digital signal through the transmitter 6 in wireless mode, if it is pushed and the finger of the left hand is not in contact with the relative sensor 21.

A red LED 7 connected to the microprocessor 5 is mounted on the circuit card 4. During detection of the ECG signal by the sensors 22, 22', the LED 7 flashes. When the ECG tracing has been acquired and stored in the memory 51, the LED 7 ceases to flash and switches out. The LED 7 also flashes during transmission in wireless mode of the stored ECG digital signal.

The microprocessor 5 which manages all the electronics of the device 100 is of the low power consumption type and comprises and comprises AD input for digitalization of the ECG signal, a port for management of the external memory 51 and a plurality of IN/OUT pins for flashing of the LED 7, for the ON/transmission pushbutton 43 and for the IRDA transmitter 6.

All the electronics of the device 100 are powered by a battery 8 mounted above the circuit card 4, inside the top shell 2. The battery 8 can be a 3V CR2032 type lithium battery commonly available commercially.

Numerous changes and modifications of detail within the reach of a person skilled in the art can be made to the present embodiment of the invention without departing from the scope of the invention as set forth in the appended claims.

## Claims

1. A device (100) for acquiring, recording and transmitting electrocardiographic (ECG) signals in relation to homecare needs, comprising a box (1, 2) containing a printed circuit card (4) on which are mounted a processor (5), a memory (51) and a transmission device (6), there being formed in said box two recessed seats (21, 21') wherein are disposed two sensors (22, 22') able to detect the electrical signal indicative of the ECG by means of contact of a finger of the right hand and of a finger of the left hand of the patient, said sensors (22, 22') being connected electrically to said processor (5) to store in said memory (51) the ECG data detected and to transmit said ECG data, by means of said transmitter (6), to a doctor's remote acquisition device, for consultation thereof.

2. A device (100) according to claim 1, **characterized in that** said sensors (22, 22') are electrodes able to detect the electrical signal generated by the heart's activity.

3. A device (100) according to claim 1 o 2, **characterized in that** it comprises an amplifier (4) and a filter (41) to amplify and filter the electrical signal detected by said sensors (22, 22') and an analog-to-digital converter (50) to transform the analogical signal detected by said sensors (22, 22') into digital signal so as to be able to store it in said memory (51).

4. A device (100) according to any one of the preceding claims, **characterized in that** said transmitter (6) is an infrared transmitter, operating with the IRDA protocol.

5. A device (100) according to any one of claims 1 to 3, **characterized in that** said transmitter (6) is a wireless transmitter, operating with any one of the following protocols: WI-FI, Bluetooth, GSM or broadband telephony, to be able to transmit said data from the memory (51) to a physician's telephone or computer.

6. A device (100) according to any one of the preceding claims, **characterized in that** it is connected by wireless to a gateway able to perform other homecare functions, including video monitoring or voice connection, so that the ECG tracing recorded by the device (100) is transmitted to said gateway which in turn transmits it, via Intranet or via Internet, to a client on which the doctor can observe it and possibly intervene.

7. A device (100) according to any one of the preceding claims, **characterized in that** it comprises a switch (43) mounted on said circuit card (4) and connected to the microprocessor (5) having the dual function of enabling the device (100) for detection and storage of the ECG signal and enabling of transmission of the data contained in the memory (51), through the transmitter (6).

8. A device (100) according to claim 7, **characterized in that** said transmission enabling switch (43) is a membrane switch disposed beneath one (22') of said two sensors and operated by pressure of said sensor (22') by the patient for a pre-set time.

9. A device (100) according to any one of the preceding claims, **characterized in that** it comprises a LED (7) mounted on said circuit board (4), connected to said microprocessor (5), and in register with a window of said box to be seen from the outside, said LED (7) remaining on for the time necessary to allow detection of the ECG signal and storage thereof in the memory (51).
